# EUROPEAN PATENT SPECIFICATION

(11) **EP 3 206 027 B1**
(45) Date of publication and mention of the grant of the patent: **11.09.2019**
(21) Application number: 16155356.5
(22) Date of filing: 11.02.2016
(51) Int. Cl.: G01N 33/00, H01L 27/02

(54) **SENSOR CHIP COMPRISING ELECTROSTATIC DISCHARGE PROTECTION ELEMENT**
SENSOR-CHIP MIT EINEM SCHUTZELEMENT GEGEN ELEKTROSTATISCHE ENTLADUNG
PUCE DE CAPTEUR COMPRENANT UN ÉLÉMENT DE PROTECTION CONTRE LES DÉCHARGES ÉLECTROSTATIQUES

(43) Date of publication of application: 16.08.2017
(73) Proprietor: Sensirion AG, 8712 Stäfa (CH)
(72) Inventor: Brugger, Thomas, 8712 Stäfa (CH); Graf, Markus, 8712 Stäfa (CH); Braun, Stephan, 8712 Stäfa (CH); Böller, Matthias, 8712 Stäfa (CH); Bartsch, Ulrich, 8712 Stäfa (CH); Vanha, Ralph Steiner, 8712 Stäfa (CH)
(74) Representative: Toleti, Martin

(56) References cited:
- DE-A1-102007 029 720
- FR-A1- 3 014 094
- GB-A- 2 484 339
- US-A- 5 407 854
- US-A1- 2008 150 050
- US-B2- 6 714 392
- DATABASE WPI Week 201282 Thomson Scientific, London, GB; AN 2012-Q98785 XP002760519, & JP 2012 242253 A (HORIBA LTD) 10 December 2012 (2012-12-10)

## Description

### Technical Field

The present idea refers to a sensor device.

### Background Art

Subject to the application, sensors tend to be integrated into chips. This kind of manufacturing is beneficial in that the size of a sensor can significantly be reduced compared to a discrete type sensor and a sensing element of such a sensor can be arranged next to electronic circuitry integrated into the very same chip which circuitry may include functions acting on a signal delivered by the sensing element such as amplification, evaluation, etc.

US20080150050 discloses an information sensing device including a chip surface to sense an image of a fingerprint, said device furthermore provided with an electrostatic protection structure.

GB 2484339 A refers to a device to detect a species in a fluid sample which comprises an electrostatic discharge (ESD) protection structure, an ion sensitive field effect transistor (ISFET) having a floating gate structure and a sensing layer located above the floating gate. The device is configured such that the electrical impedance from the sensing layer to the ESD structure is less than that from the sensing layer to the floating gate. In another embodiment the device comprises a substrate and multi-layered stratum comprising a sensing layer, a metal layer forming an ESD protection structure and one or more metal layers forming a floating gate structure, where the ESD structure is at a layer between the floating gate and the sensing layer. The device may be fabricated in a standard CMOS process and the ESD structure can form a closed loop track or ring shape connected to a protection circuit.

JP 2012-242253 A refers to an ISFET sensor including a FET, an ion sensitive layer covering a gate of the FET, an ESD protection layer coating the parts other than the gate part, and an external protection circuit connected to the ESD protection layer having an ESD protection element. The impedance of circuits constituted of the FET is set to be greater than the impedance of the external protection circuit.

DE 10 2007 029720 A1 refers to a simple, space-saving and cost-effective construction of a housing for an ASIC, wherein the housing has terminals for connecting to the ASIC from the outside via electrical lines. At least one electronic component is provided to protect the ASIC against electrostatic discharge (ESD) and to suppress EMC voltages induced via the lines. The at least one component is arranged inside the housing and is connected to at least one of the terminals.

US 6,714,392 B2 describes an electronic component which has a dielectric layer on a substrate, conductive surfaces that are constructed on the dielectric layer, and an electrically conductive guard structure. The guard structure is disposed in a plane above the conductive surfaces such that the conductive surfaces are not completely covered by the guard structure.

US 5,407,854 A refers to methods, apparatus and chip fabrication techniques which provide electrostatic discharge (ESD) protection to ion-sensitive field effect transistor (ISFET) based devices used to selectively measure ions in a liquid. An ESD protection circuit, made up of conventional protective elements, is integrated onto the same silicon chip on which the ISFET is formed, along with an interface that is in contact with the liquid being measured and which does not open up paths for D.C. leakage currents between the ISFET and the liquid. A capacitor structure is used as the interface between the protection circuit and the liquid sample.

FR 3 014 094 A1 relates to a micro-machined sensor (MEMS or NEMS). The sensor uses a strain gage detection and is formed on a chip (IC) comprising a substrate and external connection pads. The sensor comprises at least one strain gauge made in a layer of piezo-resistive material and located at a distance of several hundred micro-meters from a connection pad. An array of conductors connects the connection pads to the micro-machined sensor. At least one impedance matching resistor is made in the same layer of piezoresistive material as the strain gauge and is placed in close proximity of the strain gauge.

### Disclosure of the Invention

It is a general object of the invention to provide a sensor device with an improved protection.

A sensor device according to the features of claim 1 comprises a chip and a sensing element. The chip preferably is a semiconductor chip comprising a semiconductor substrate such as a silicon substrate, and preferably comprising one or more material layers on the substrate, such as one or more of a metal layer, an insulating layer, a passivation layer. The side of the chip at which the one or more material layers are arranged is denoted as top side of the chip. Preferably, this top side may contain active circuitry such as a processing circuit for processing signals supplied by the sensing element. Such active circuitry may be integrated into the chip, e.g. by conventional CMOS processing.

The sensing element preferably is either arranged on the chip, or is integrated therein. This may include any mounting, arrangement, fixing, depositing or growing of the sensing element at the top side of the chip which may be on top of the substrate or on top of the material layers if any. The sensing element in one embodiment may be an element separate from the chip and be attached thereto. An integration of the sensing element into the chip from the top side may include the manufacturing of sensing element structures in the substrate of the chip or in any of the material layers possibly deposited onto a substrate of the chip. Specifically, the sensing element may be formed by a doped region in a semiconductor substrate of the chip.

In a preferred embodiment, the sensing element may comprise a sensitive layer, and preferably at least two electrodes. The sensing element is sensitive to one or more of a chemical analyte, humidity, a flow of a fluid, a pressure, light or temperature.

The sensing element or the chip as such may be exposed to an electrostatic discharge which in turn may damage the sensing element and / or the chip. In order to prevent such damage, an electric discharge protection element is provided. This results in an effective protection of one or more of the sensing element, the circuitry if any, and the chip as such from electric discharge.

In a preferred embodiment, the protection element is arranged on the chip at its top side, or is integrated in the chip. This may include any mounting, arrangement, fixing, depositing, and in particular vapor depositing or growing of the protection element on top of the substrate or on top of the material layers if any. The protection element in one embodiment may be an element separate from the chip and be attached thereto. However, any building of a protection element from elements already present in the chip shall be included. For example, one or more CMOS layers possibly deposited onto a substrate as material layers of the chip may contribute to the protection element.

In a very preferred embodiment, the protection element is an electrically conductive structure, and in particular is a metallic structure. A deposition of the protection element may vary subject to the location and the surface the protection element is applied to and subject to the material used for the protection element: In one embodiment, the protection element is sputtered onto the chip. In another embodiment, the protection element is applied as a conductive adhesive, and in particular as a conductive epoxy resin. In a further embodiment, the protection element may be applied by photolithography.

Whenever a CMOS process is applied for manufacturing the chip, parts of one or more metallization layers, preferably close to the area of the sensing element, may serve as protection element and as such serve as an entrance for an electric discharge. Hence, in one embodiment, a part of such metallization layer may contribute to the protection element.

The protection element preferably encircles the sensitive element, e.g. in form of a ring of circular or rectangular or any other shape. The ring may be closed while in a different embodiment, the ring is interrupted, i.e. not closed.

Or, the protection element is arranged on top of the sensing element provided the sensing element is exposed. This may especially be preferred if the sensitive element comprises a sensitive layer that is arranged on the chip.

In a different embodiment, the protection element may include a metallic wire protruding from the chip. Specifically, the metallic wire may be a bond wire. Especially in case of a bond wire, the protection element spans the sensing element without touching it. In another embodiment, the protection element comprises a stud bump. Such protection element is arranged on the sensing element itself.

Preferably, the protection element is provided in addition to electrodes for electrically connecting the sensor element. Preferably, the protection element provides a lowest electrical resistance out of the metallic elements that are provided.

The sensor device comprises a package for the chip. Preferably, the package of the sensor device is a package that protects the chip from mechanical impact and light, and may seal the chip against an undesired impact of liquids or gases. In a preferred embodiment, the package may cover at least parts of the chip in form of an encapsulation. However, given that the sensing element is a sensing element requiring access to the outside of the sensor device for measuring one or more parameters of the environment, such as chemical substances, humidity, pressure, or the flow of a fluid, such sensing element is desired to be accessible from the outside such that the medium to be measured may access/get in touch with the sensing element. Whenever the package encloses the chip an access opening may take the form of a recess in the package. Preferably, the package contains a mould compound covering at least part of the chip, while the access opening may be represented by a recess in the mould compound. Preferably, the protection element is at least located in the access opening and is exposed to the outside of the sensor device, e.g. in an area of the chip uncovered by the package and as such is accessible from the outside such that an electric discharge can be trapped from the protection element. Preferably, the chip may include through silicon vias. In another embodiment, the chip may be flip-chip arranged onto a carrier with the carrier providing an opening for granting access to the sensing element. In another embodiment, the package may represent a QFN - quad-pads no-leads - package.

In a preferred embodiment, the sensor device, comprises an electrical drain connection for draining electrical charges trapped by the protection element. In one embodiment, the drain connection may be a ground connection. Specifically, the protection element is electrically connected to the substrate of the chip which may be operated on ground potential. In another embodiment, the drain connection may include an electrically conducting path within the chip towards a contact pad of the chip out of a set of contact pads to which contact pad ground potential is applied during operation. The electrically conducting path may be a part of a metal layer of the stack of material layers, and / or a via through the substrate, for example. In a further embodiment, the sensor device includes a carrier for the chip such as a leadframe. Here, the protection element may directly be connected to a contact pad of the carrier to which contact pad ground potential may be applied during operation. The drain connection between the protection element and the contact pad of the carrier may, for example, be a bond wire.

In case a carrier is provided in the sensor device for holding the chip and for allowing an electrical connection to the chip, and in case the sensor device comprises a package for enclosing or encapsulating the chip, the carrier, such as a leadframe may at least partially be encapsulated, too.

This present idea results in an effective protection of the sensing element and the circuitry if any from electric discharge e.g. supplied through an access opening in a package of the sensor device.

Other advantageous embodiments are listed in the dependent claims as well as in the description below.

### Brief Description of the Drawings

The embodiments defined above and further embodiments, features and advantages of the present invention can also be derived from the examples of embodiments to be described hereinafter in connection with the drawings in which the figures illustrate:

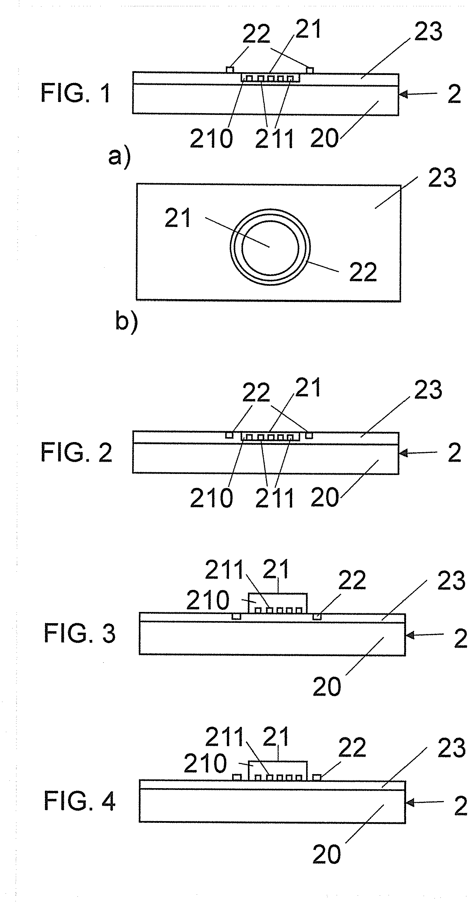

FIGs. 1 - 13 each a lateral cut of a sensor device according to an embodiment of the present invention, and additionally a top view in diagram 1b).

### Modes for Carrying Out the Invention

FIG. 1 illustrates a sensor device according to an embodiment of the present invention. According to the side cut in diagram 1a), the sensor device contains a chip 2, for example a semiconductor chip with a substrate 20, such as a silicon substrate, and a stack of material layers 23 such as a CMOS layer stack on top of the substrate 20. The side of the chip 2 at which the material layers 23 are arranged at is referred to as top side. A sensing element 21 is integrated into the chip 2, i.e. arranged in the stack of material layers 23 in the present example. The sensing element 21 comprises at least two electrodes, such as interdigitated electrodes, collectively referred to as 211. A sensitive layer 210 covers the electrodes 211. An electric discharge protection element 22 is arranged on the chip 2 at its top side in form of a ring encircling the sensing element 21 in a horizontal plane of the chip 2. This can be derived from the top view illustrated in diagram 1b). "Encircling" and "ring" do not necessarily imply a closed and circular structure in this and any of the other embodiments. The ring shape may also contain rectangles, triangles, ellipses, etc., and / or may also include non-continuous structures, e.g. a ring shape including interrupts as long as the resulting individual elements of the ring are properly drain connected.

It is noted that the present sensor device as well as the sensor devices introduced in the following Figures 2 to 11 additionally comprises a package such as an encapsulation or a housing which comprises an access opening to the sensing element. Then, the protection element preferably is arranged at least partly exposed to the outside via the access opening.

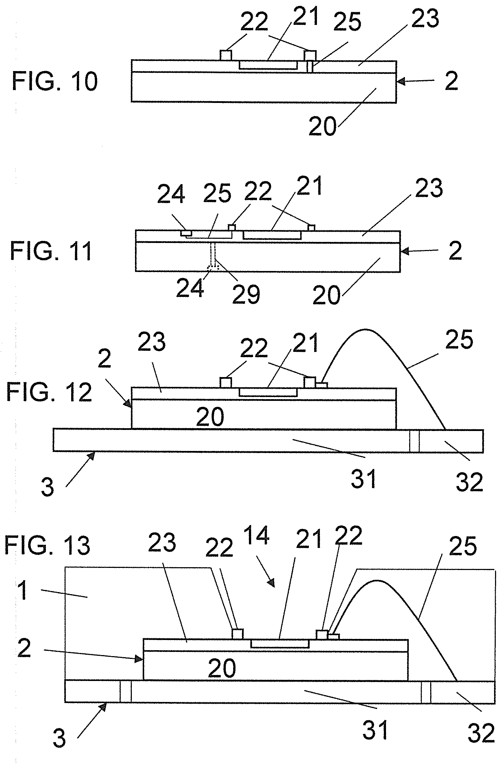

In addition, it is noted that the protection element preferably is electrically connected to a drain connection for draining an electric discharge if any, which drain connection is not explicitly shown in the Figures 1 to 9 for illustration purposes but is referred to in more detail in various embodiments as shown in Figures 10 to 12.

In the present and all subsequent embodiments, the protection element preferably comprises a metallic structure for protecting the sensor device from electrostatic discharge.

FIG. 2 illustrates a sensor device in accordance with another embodiment of the present invention. In contrast to the embodiment of Figure 1, the protection element 22 is integrated in the chip 2, and specifically is integrated into the stack of material layers 23. However, its top surface is exposed to the outside. Preferably, the protection element 22 may be manufactured from a metal layer of the stack of material layers 23, and again may encircle the sensing element 21. In case the sensing element 21 comprises electrodes 211 such as shown in Figure 1 and Figure 2, these electrodes 211 may be manufactured from one or more metal layers of the stack of material layers 23, too. In a preferred embodiment, the protection element 22 and the electrodes 211 may be manufactured from the same metal layer of the stack of material layers 23, and hence in the same manufacturing step.

FIG. 3 illustrates a sensor device according to another embodiment of the present invention. In contrast to the embodiment of Figure 2, the sensing element 21, again e.g. containing electrodes 211 and a sensitive layer 210, now is arranged on top of the chip 2 and as such protrudes from the top side of the chip 2. The protection element 22 continues to take the form of a ring encircling the sensing element 21 and is integrated in the stack of material layers 23, again, with its top surface being exposed to the outside.

In the embodiment of FIG. 4, the sensor device differs from the sensor device of Figure 3 in that the protection element 22 is arranged on the chip 2 as is the sensing element 21. Again, the protection element 22 preferably encircles the sensing element 21. In this embodiment, the electrodes 211 - if any - and the protection element 22 may preferably be manufactured in the same processing step, prior to covering the electrodes by the sensitive layer 210.

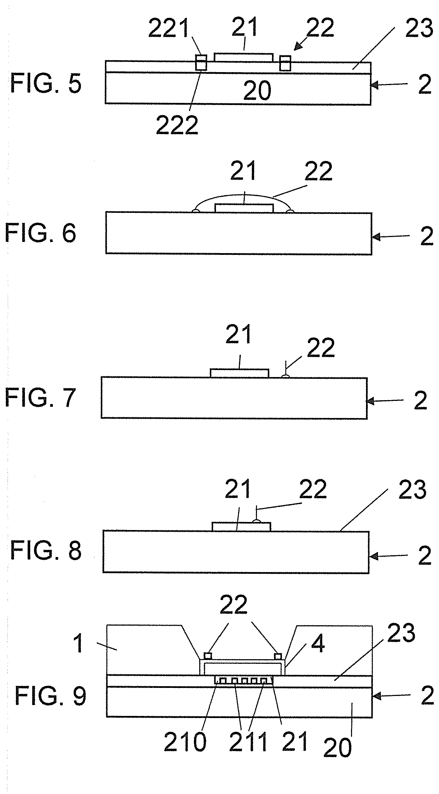

In the embodiment of FIG. 5, the protection element 22 now is at least partly integrated into the chip 2, and may be formed by a combination of one or more metal layers of the CMOS layer stack referred to by 222 and a metal ring 221 on top of the chip 2. The sensing element 21 may be arranged on the chip 2 or be integrated into the chip 2. Electrodes and the sensitive layer of the sensing element are no longer shown but may still contribute thereto.

In the embodiment of FIG. 6, the sensing element 21 is arranged on the chip 2, or may be integrated into the chip 2. The protection element 22 now is represented by a bond wire spanning the sensing element 21. Substrate and material layers of the chip are no longer shown, however, may still contribute thereto.

In the embodiment of FIG. 7, the protection element 22 is represented by a stud bump arranged on the first surface of the chip 2 next to the sensing element 21. Stud bumps can be manufactured by means of a dedicated process in which a bond wire is pulled off after being soldered with its first end.

In the embodiment of FIG. 8, the protection element 22 again is represented by a stud bump that is now arranged on the sensing element 21 itself. There may be means provided for electrically isolating the stud bump from the sensing element 21 in order not to discharge an electrical charge into the sensing element 21. The protection element 22 in Figure 8 may not necessarily be a stud bump but can also take any form or a metal deposition on the sensing element 21, and specifically on the sensitive layer of the sensing element 21, and can take any shape such as a ring or a post.

In the embodiment of FIG. 9, a frame 4 is provided on the chip 2. The frame 4 may serve for supporting the manufacturing of an encapsulation later on, or may serve as mechanical protection. In the first case, a protrusion of a mold for generating the access opening may rest on the frame during molding. A package 1 is indicated which may be manufactured with the aid of such frame 4. The protection element 22 in this instance is arranged on the frame 4 and via the frame 4 on the chip 2. Again, the protection element 22 may take the shape of a ring or a post.

Figures 10 to 12 illustrate embodiments of a sensor device which may comprise a sensing element, its arrangement, a protection element and its arrangement according to any of the previous embodiments. Focus is now on the drain connection that preferably is provided for draining an electrical discharge trapped by the protection element. In the sensor device of Figure 10, the protection element 22 is electrically connected to the substrate 20 of the chip 2 - which preferably is semiconductor substrate 20 - by means of a drain connection 25 in form of a via through the stack of the material layers 23. The drain connection 25 preferably is electrically isolated from other electrical paths in this stack. The substrate 20 preferably is operated on ground potential and therefore serves for draining an electrical discharge captured by the protection element 22. In a preferred embodiment, the chip 2 is arranged on a carrier (not shown), and specifically is arranged on a die pad of the carrier and is electrically connected thereto with its substrate 20 for further draining an electrical discharge.

Figure 11 illustrates another embodiment of a drain connection that is provided for draining an electrical discharge. A contact pad 24 out of a set of contact pads 24 arranged at the top side of the chip 2 is electrically connected via a drain connection 25 to the protection element 22. The drain connection 25 preferably is built from one or more of the metal layers of the CMOS layer stack 23. A bond wire not shown may provide an electrical connection from the contact pad 24 to a carrier serving for further draining. The carrier may for example, be a leadframe the chip 2 is attached to. The bond wire may later on be encapsulated by a mould compound of the sensor device. In a different embodiment, contact pads may be provided at the backside of the substrate, including contact pad 24 indicated by dots, and a silicon through via indicated by reference numeral 29 may contribute to connecting the protection element 22 to the contact pad 24 as a drain connection. This concept may also be applied to any of the other embodiments.

According to the embodiment of Figure 12, the chip 2 is arranged on a carrier 3 in form of a leadframe comprising a die pad 31 for holding the chip 2 and contact pads 32 electrically isolated from each other. In this example, the protection element 22 is directly connected to the carrier 3 via the drain connection 25 in form of a bond wire, and specifically to a contact pad 32 of the carrier 3 which may be operated at ground potential. Hence, the drain connection is completely outside the chip 2. The bond wire may later on be encapsulated by a mould compound of the sensor device.

FIG. 13 illustrates another sensor device according to an embodiment of the present invention. Here, the chip 2 and the carrier 3 of Figure 11 are packaged by a package 1. The package 1 contains an access opening 14 for granting access to a sensing element 21. The protection element 22 is arranged within the access opening 14 and is electrically connected via the drain connection 25 in form of the bond wire to the contact pad 32 of the carrier 3. This electrical path preferably represents the ground connection.

The package 1 may preferably be formed by molding or casting a mould compound onto the chip wherein an insert is provided in the mold for forming the access opening above the sensing element. The material used may preferably be a resist, in particular a dry resist, for example SU-8. Or, the package may be an element formed separate from the chip and may be attached to the chip later on, for example, by gluing, bonding, etc. Here, the package may be a silicon-on-insulator, or other semiconducting layer arrangement. The package may be made from one of a semiconductor, silicon, silicon and a silicon-oxide coating, silicon and a solderable coating, ceramic, ceramic and a silicon-oxide coating, ceramic and a solderable coating, glass, glass and a silicon-oxide coating, glass and a solderable coating, metal, metal and a solderable coating, dielectric material and a polymer. The package may cover and protect at least part of circuitry, and in particular metal structures, integrated into the substrate. However, it is preferred that the package does not cover the sensing element 21.

While there are shown and described presently preferred embodiments of the invention, it is to be distinctly understood that the invention is not limited thereto but may be otherwise variously embodied and practiced within the scope of the following claims.

## Claims

1. Sensor device, comprising
a chip (2),
a sensing element (21),
a package (1) for the chip (2) with an access opening (14) for providing access to the sensing element (21) from the outside of the sensor device, and
an electrostatic discharge protection element (22) for protecting the sensing element (21) and / or the chip (2) from an electrostatic discharge, **characterized in that**
the protection element (22) is one of:
- is arranged on the sensing element (21),
- spans the sensing element (21), or
- encircles the sensing element (21),
wherein at least a part of the protection element (22) is arranged exposed to the outside via the access opening (14), and
wherein the sensing element (21) is adapted to sense at least one of a chemical analyte, humidity, a flow of a fluid, a pressure.

2. Sensor device according to claim 1,
wherein the sensing element (21) is arranged on the chip (2), or
wherein the sensing element (21) is integrated into the chip (2).

3. Sensor device according to any one of the preceding claims,
wherein the protection element (22) is arranged on the chip (2), or
wherein the protection element (22) is integrated into the chip (2).

4. Sensor device according to any one of the preceding claims,
wherein the sensing element (21) comprises at least two electrodes (211) and a sensitive layer (210), and
in particular wherein the at least two electrodes (211) and the protection element (22) are arranged in a common plane.

5. Sensor device according to any one of the preceding claims,
wherein the chip (2) comprises a substrate (20), and in particular a semiconductor substrate, and one or more material layers (23) on the substrate (20), and
in particular wherein the sensing element (21) and / or the protection element (22) in case of being arranged at least partly on the chip (2) are arranged on a top surface of the one or more material layers (23).

6. Sensor device according to any one of the preceding claims,
wherein the protection element (22) is an electrically conductive structure, and in particular is one of:
- a metallic structure;
- made from a conductive adhesive, and in particular from a conductive epoxy resin.

7. Sensor device according to any one of the preceding claims,
wherein the protection element (22) comprises at least a part of a metallization layer resulting from applying a CMOS process for manufacturing the chip (2).

8. Sensor device according to any one of the preceding claims,
wherein the protection element (22) comprises a wire, and in particular a bond wire.

9. Sensor device according to any one of the preceding claims,
wherein the protection element (22) comprises a stud bump.

10. Sensor device according to any one of the preceding claims,
wherein the chip (2) comprises a set of contact pads, and
wherein the chip (2) comprises an electrical drain connection (25) connecting the protection element (22) to one of the contact pads (24) of the set, and
in particular wherein the electrical drain connection (24) is a ground connection.

11. Sensor device according to any one of the preceding claims,
wherein the protection element (22) is electrically connected to the substrate (20) of the chip (2).

12. Sensor device according to any one of the preceding claims,
comprising a carrier (3) for the chip (2), the carrier (3) comprising a contact pad (32) for a ground connection,
wherein the protection element (22) is electrically connected to the contact pad (32).

13. Sensor device according to any one of the preceding claims,
wherein circuitry (28) is integrated into the chip (2) and is electrically connected to the sensing element (21).

## Patentansprüche

1. Sensorvorrichtung, umfassend
einen Chip (2),
ein Sensorelement (21),
ein Gehäuse (1) für den Chip (2) mit einer Zugangsöffnung (14) zum Bereitstellen des Zugangs zum Sensorelement (21) von der Aussenseite der Sensorvorrichtung, und
ein elektrostatisches Entladungs-Schutzelement (22) zum Schutz des Sensorelements (21) und/oder des Chips (2) vor einer elektrostatischen Entladung, **dadurch gekennzeichnet, dass**
das Schutzelement (22) eines der folgenden ist:
- auf dem Sensorelement (21) angeordnet ist,
- das Sensorelement (21) überspannt, oder
- das Sensorelement (21) umgibt,
wobei mindestens ein Teil des Schutzelements (22) über die Zugangsöffnung (14) nach aussen freiliegend angeordnet ist, und
wobei das Sensorelement (21) angepasst ist, um mindestens eine der folgenden Grössen zu messen: einen chemischen Analyten, Feuchtigkeit, Durchfluss eines Fluids, Druck.

2. Sensorvorrichtung gemäss Anspruch 1,
wobei das Sensorelement (21) auf dem Chip (2) angeordnet ist, oder
wobei das Sensorelement (21) in den Chip (2) integriert ist.

3. Sensorvorrichtung gemäss einem der vorstehenden Ansprüche,
wobei das Schutzelement (22) auf dem Chip (2) angeordnet ist, oder
wobei das Schutzelement (22) in den Chip (2) integriert ist.

4. Sensorvorrichtung gemäss einem der vorstehenden Ansprüche,
wobei das Sensorelement (21) mindestens zwei Elektroden (211) und eine empfindliche Schicht (210) umfasst, und
insbesondere wobei die mindestens zwei Elektroden (211) und das Schutzelement (22) in einer gemeinsamen Ebene angeordnet sind.

5. Sensorvorrichtung gemäss einem der vorstehenden Ansprüche,
wobei der Chip (2) ein Substrat (20), insbesondere ein Halbleitersubstrat, und eine oder mehrere Materialschichten (23) auf dem Substrat (20) umfasst, und
insbesondere wobei das Sensorelement (21) und/oder das Schutzelement (22), wenn sie zumindest teilweise auf dem Chip (2) angebracht sind, auf einer Oberseite der einen oder mehreren Materialschichten (23) angeordnet sind.

6. Sensorvorrichtung gemäss einem der vorstehenden Ansprüche,
wobei das Schutzelement (22) eine elektrisch leitfähige Struktur ist und insbesondere eines von:
- eine metallische Struktur;
- hergestellt aus einem leitfähigen Klebstoff, insbesondere aus einem leitfähigen Epoxidharz.

7. Sensorvorrichtung gemäss einem der vorstehenden Ansprüche,
wobei das Schutzelement (22) mindestens einen Teil einer Metallisierungsschicht umfasst, die von der Anwendung eines CMOS-Prozesses zur Herstellung des Chips (2) stammt.

8. Sensorvorrichtung gemäss einem der vorstehenden Ansprüche,
wobei das Schutzelement (22) einen Draht und insbesondere einen Bonddraht umfasst.

9. Sensorvorrichtung gemäss einem der vorstehenden Ansprüche,
wobei das Schutzelement (22) einen Stud-Bump umfasst.

10. Sensorvorrichtung gemäss einem der vorstehenden Ansprüche,
wobei der Chip (2) ein Set von Kontaktflächen umfasst, und
wobei der Chip (2) eine elektrische Drainverbindung (25) umfasst, die das Schutzelement (22) mit einer der Kontaktflächen (24) des Sets verbindet, und
insbesondere wobei die elektrische Drainverbindung (24) eine Masseverbindung ist.

11. Sensorvorrichtung gemäss einem der vorstehenden Ansprüche,
wobei das Schutzelement (22) elektrisch mit dem Substrat (20) des Chips (2) verbunden ist.

12. Sensorvorrichtung gemäss einem der vorstehenden Ansprüche,
umfassend einen Träger (3) für den Chip (2), wobei der Träger (3) eine Kontaktfläche (32) für eine Masseverbindung umfasst,
wobei das Schutzelement (22) elektrisch mit der Kontaktfläche (32) verbunden ist.

13. Sensorvorrichtung gemäss einem der vorstehenden Ansprüche,
wobei ein Schaltkreis (28) in den Chip (2) integriert und elektrisch mit dem Sensorelement (21) verbunden ist.

## Revendications

1. Dispositif capteur, comprenant
une puce (2),
un élément de détection (21),
un boîtier (1) pour la puce (2) avec une ouverture d'accès (14) pour permettre l'accès à l'élément de détection (21) depuis l'extérieur du dispositif capteur, et
un élément de protection contre les décharges électrostatiques (22) pour protéger l'élément de détection (21) et/ou la puce (2) contre une décharge électrostatique, **caractérisé en ce que**
l'élément de protection (22) est l'un parmi:
- est disposé sur l'élément de détection (21),
- s'étend sur l'élément de détection (21), ou
- entoure l'élément de détection (21),
au moins une partie de l'élément de protection (22) étant disposée de manière exposée vers l'extérieur via l'ouverture d'accès (14), et
l'élément de détection (21) étant adapté pour détecter au moins l'un parmi un analyte chimique, l'humidité, un écoulement d'un fluide, une pression.

2. Dispositif capteur selon la revendication 1,
l'élément de détection (21) étant disposé sur la puce (2), ou
l'élément de détection (21) étant intégré dans la puce (2).

3. Dispositif capteur selon l'une des revendications précédentes,
l'élément de protection (22) étant disposé sur la puce (2), ou
l'élément de protection (22) étant intégré dans la puce (2).

4. Dispositif capteur selon l'une des revendications précédentes,
l'élément de détection (21) comprenant au moins deux électrodes (211) et une couche sensible (210), et
en particulier les au moins deux électrodes (211) et l'élément de protection (22) étant disposés dans un plan commun.

5. Dispositif capteur selon l'une des revendications précédentes,
la puce (2) comprenant un substrat (20), et en particulier un substrat semi-conducteur, et une ou plusieurs couches de matériau (23) sur le substrat (20), et
en particulier l'élément de détection (21) et/ou l'élément de protection (22) en cas de disposition au moins partielle sur la puce (2) étant disposés sur une surface supérieure de la ou des couches de matériau (23).

6. Dispositif capteur selon l'une des revendications précédentes,
l'élément de protection (22) étant une structure électriquement conductrice, et en particulier un élément parmi:
- une structure métallique;
- fait à partir d'un adhésif conducteur, et en particulier d'une résine époxy conductrice.

7. Dispositif capteur selon l'une des revendications précédentes,
l'élément de protection (22) comprenant au moins une partie d'une couche de métallisation résultant de l'application d'un procédé CMOS pour fabriquer la puce (2) .

8. Dispositif capteur selon l'une des revendications précédentes,
l'élément de protection (22) comportant un fil, et en particulier un fil de liaison.

9. Dispositif capteur selon l'une des revendications précédentes,
l'élément de protection (22) comprenant une bosse de goujon.

10. Dispositif capteur selon l'une des revendications précédentes,
la puce (2) comprenant un jeu de pastilles de contact, et
la puce (2) comprenant une connexion de drain électrique (25) reliant l'élément de protection (22) à l'une des plages de contact (24) de l'ensemble, et
en particulier la connexion de drain électrique (24) étant une connexion de masse.

11. Dispositif capteur selon l'une des revendications précédentes,
l'élément de protection (22) étant connecté électriquement au substrat (20) de la puce (2).

12. Dispositif capteur selon l'une des revendications précédentes,
comprenant un support (3) pour la puce (2), le support (3) comprenant un plot de contact (32) pour une connexion de masse,
l'élément de protection (22) étant connecté électriquement au plot de contact (32).

13. Dispositif capteur selon l'une des revendications précédentes,
des circuits (28) étant intégrés dans la puce (2) et étant connectés électriquement à l'élément d'émission (21).
